# EUROPEAN PATENT SPECIFICATION

(11) **EP 0 222 718 B1**
(45) Date of publication and mention of the grant of the patent: **24.11.1993**
(21) Application number: 86850350.9
(22) Date of filing: 14.10.1986
(51) Int. Cl.: C08J 3/12, C08J 9/28, B01D 15/08, B01J 20/26, C12N 5/00

(54) **Macroporous particles**
Makroporöse Partikel
Particules macroporeuses

(30) Priority: 15.10.1985 SE 8504764
(43) Date of publication of application: 20.05.1987
(73) Proprietor: Nilsson, Kjell G.C., S-222 51 Lund (SE); MOSBACH, Klaus H., S-244 02 Furulund (SE)
(72) Inventor: Nilsson, Kjell G.C., S-222 51 Lund (SE); MOSBACH, Klaus H., S-244 02 Furulund (SE)
(74) Representative: Larsson, Karin

(56) References cited:
- EP-A- 0 087 786
- DE-A- 1 959 649
- DE-A- 2 035 601
- GB-A- 2 061 954

## Description

The present invention concerns macroporous particles, processes to produce these particles and the use of the particles as ion exchanger in gel filtration, hydrophobic chromatography, affinity chromatography or as microcarriers in cell cultivation processes.

The separation of molecules is effected to a large extent with the aid of matrices which have connected thereto ligands which interact with the molecules concerned. These ligands may be ionic, hydrophobic or affinity ligands. Electrically neutral matrices of mutually different porosity are used when separating molecules in accordance with size, i.e. gel filtration. These matrices are normally spheroidal in shape, in order to afford good flow properties. The flow properties of the separation system are also determined by the size of the particles present; the smaller the particles the higher the pressure drop, which results in a lower rate of flow. It is desirable in industrial applications to achieve high rates of flow, so that the molecules can be separated quickly. Another important parameter with regard to the particles used is the total specific surface area presented by the particles. The larger the specific surfaces are the more quickly the molecules are able to penetrate the matrix and interact with the ligands. The specific surface area can be increased by reducing the sizes of the particles.

This antithesis is usually solved by taking a middle path, i.e. by using a relatively large particle size which is not optimum with regard to either the flow properties of the separation system or the specific surface area.

The present invention overcomes the antithesis by manufacturing particles which enclose a large number of cavities, so that the particles can be given a size which while enabling a high rate of flow to be achieved also present a very large specific surface area. In addition to the separation of molecules, the extremely large surface area of the particles provided enables the particles to the used for cultivating anchorage dependent cells. In this technique, the anchorage dependent cells are allowed to grow on the surfaces of particles, microcarriers, suspended in a nutrient. Because the interiors of the particles can now also be used in cell cultivation processes, the surface area available is much larger and mechanical protection is also afforded to the cells during the process of cultivation.

Thus, the invention concerns macroporous particles which are characterized in that they have a particle size of 10-500 µm and consist of a polymer matrix enclosing a large number of cavities having a diameter of 1-50 µm, which particles have been produced by mixing a solution of a matrix material, prepared by dissolving a polymer and/or polymerizable monomers in an aqueous solvent for the matrix material and addition of a water-soluble emulsifier, with a solid cavity generating compound to make a suspension of the solid compound in the solution; forming particles of the polymer matrix by dispersion of the suspension in a water-insoluble dispersion medium containing a water-insoluble emulsifier and solidifying the suspension droplets obtained in the medium; and washing out the solid cavity generating compound from the particles.

The invention also concerns macroporous particles which are caracterized in that they have a particle size of 10-500 µm and consist of a polymer matrix enclosing a large number of cavities having a diameter of 1-50 µm, which particles have been produced by mixing a solution of a matrix material, prepared by dissolving a polymer and/or polymerizable monomers in an aqueous solvent for the matrix material and addition of a water-soluble emulsifier, with a water-insoluble dispersion medium containing a water-insoluble emulsifier to make a dispersion of the medium in the solution; forming particles of the polymer matrix by dispersing the dispersion in a further amount of the dispersion medium and solidifying the dispersion droplets obtained in the medium; and washing out the dispersion medium from the particles.

These macroporous particles are produced by
(A) dissolving a matrix material, selected from a water-soluble polymer and/or polymerizable monomers, in an aqueous solvent for the matrix material;
(B) adding a water-soluble emulsifier;
(C) forming cavity containing droplets of the matrix material by:
   a) adding a solid cavity generating compound which is insoluble in the aqueous solution while stirring the system to form a suspension of the cavity generating compound in the aqueous solution; and
   b) adding the suspension to a water-insoluble dispersion medium containing a water-insoluble emulsifier which dissolves in the dispersion medium while stirring to form a dispersion of droplets of the suspension in the dispersion medium;
D) solidifying the dispersed droplets by cooling, covalent crosslinking or by polymerizitation to form beads of the polymer matrix each bead having a large number of pores containing the solid cavity generating compound; and
E) washing out the solid cavity generating compound from the pores of the beads to form the macroporous particles with a large number of cavities;
or by
(A) dissolving a matrix material, selected from a water-soluble polymer and/or polymerizable monomers, in an aqueous solvent for the matrix material;
(B) adding a water-soluble emulsifier;
(C) forming cavity containing droplets of the matrix material by:
   a) adding a liquid cavity generating material consisting of a water-insoluble dispersion medium containing a water-insoluble emulsifier which dissolves in the dispersion medium, while stirring the system until the dispersion medium forms droplets in the aqueous solution and an emulsion is obtained; and
   b) adding additional dispersion medium to saturate the aqueous solution with the dispersion medium and to form droplets of the emulsion in the dispersion medium;
D) solidifying the dispersed droplets by cooling, covalent crosslinking or by polymerizitation to form beads of the polymer matrix each bead having a large number of pores containing the dispersion medium ; and
E) washing out the dispersion medium from the pores of the beads to form the macroporous particles with a large number of cavities.

Subsequent to the removal of the solid or liquid cavity generating compound, the resultant microporous particles can either be derivatized or used directly for their intended purposes.

The matrix forming compound is selected from proteins, polysaccharides or synthetic polymers. Examples of compounds which can be used are:
proteins - gelatin, albumin
polysaccharides - dextran, agarose
synthetic polymers - polyacrylamide.

An example of a solid cavity generating compound is calcium carbonate, which after the particles have been produced by dispersing the mixture in a water-insoluble dispersion medium and the matrix has been made insoluble in water, can be removed by treating the system with an acid.

When the cavity generating compound used is in liquid form, it is necessary to add emulsifiers. The aqueous solution of the matrix is admixed with a water soluble emulsifier (characterized by an HLB-value greater than 9). Droplets of the cavity generating compound are formed by continuously adding the liquid cavity generating compound admixed with a water-insoluble emulsifier (characterized by a HLB-value lower than 8) to the aqueous solution of the matrix while stirring the system; the more vigorous the agitation the smaller the droplets formed. When the cavity generating compound has been added in an amount sufficient to saturate the aqueous solution of the matrix material, further addition will cause the matrix solution to form droplets in the excess of the cavity generating compound. By selecting emulsifiers which result in stable dispersions, particles of the matrix material which contain droplets of the cavity generating compound are obtained, subsequent to rendering the matrix insoluble in water. The cavity generating compound is then washed out with a solvent. The majority of organic solvents (water insoluble) can be used as the liquid cavity generating compound, as can also vegetable oils or mineral oils. Examples of suitable emulsifiers are Span® 85, Arlacel® 83 (water insoluble) and Tween® 80, Triton® X-100 (water soluble).

### Example 1

### Thermal gelation (liquid cavity generating compound).

Gelatin was dissolved by heating the same in water to a concentration of 10 % (w/v). 6 g of emulsifier (Tween® 80) were added to 100 ml of the gelating solution. 500 ml of toluene containing 30 g emulsifier (Span® 85) were then stirred into the solution. When beads of the desired size had formed, the dispersion was cooled to a temperature beneath the solidification temperature of the gelatin. The aforedescribed process results in the formation of gelatin beads which are saturated with droplets of toluene. These toluene droplets can be removed by washing the beads with ethanol and acetone, therewith providing a gelatin bead which is filled with cavities.

The gelatin beads can then be cross-linked with, for example, glutardialdehyde, in order to further increase stability.

### Example 2

### Thermal gelation (solid cavity generating compound).

10 g of calcium carbonate were added to 100 ml of gelatin solution (10 % w/v), whereafter beads were produced in accordance with Example 1. The beads were treated with acid, so as to dissolve the calcium carbonate and therewith form cavities in the beads.

### Example 3

### Polymerization.

Acrylamide (17 g) and bisacrylamide (1.2 g) were dissolved in a Tris-buffer (100 ml 0.05M, pH 7). Ammonium persulphate (0.5 g/ml, 0.25 ml) and emulsifier (Triton® x-100, 6 g) were added to the monomer solution. 500 ml of toluene containing an emulsifier (Span® 85, 30 g) were then stirred into the system. TEMED (co-catalyst, 1.3 ml) was then added to the system. The organic solvents were washed out with ethanol and acetone, upon termination of the polymerization process.

### Example 4

### Covalent cross-linking.

Sodium hydroxide (0.7 g) and emulsifier (Tween® 80, 6 g) were added to an aqueous solution of dextran (10 %, w/v, 100 ml). Toluene (500 ml) having an emulsifier (Span® 85, 30 g) and epichlorohydrin (1.5 g) dissolved therein was then added to the solution while stirring the system. The temperature of the system was raised to 40°C over a period of 2 hours, and then to 70°C over a further period of 12 hours. The resultant beads were washed with ethanol and acetone, in order to remove organic solvent. The properties of the beads formed can be varied, by varying the quantity of dextran and the quantity of epichlorohydrin used.

### Example 5

### Covalent cross-linking.

Chitosan was dissolved in formic acid (5 %, w/v) to a concentration of 30 g/l. 100 ml of solution were admixed with emulsifier (Tween® 80, 6 g) and, while stirring the system, with toluene (500 ml) containing an emulsifier (Span® 85, 30 g). Subsequent to obtaining beads of the desired size, formaldehyde (20 ml) was added to the system. The resultant beads were washed with methanol after a time lapse of one hour.

## Claims

1. Macroporous particles to be used as ion exchanger in gel filtration, hydrophobic chromatography, affinity chromatography or as microcarriers in cell cultivation processes, characterized in that they have a particle size of 10-500 µm and consist of a polymer matrix enclosing a large number of cavities having a diameter of 1-50 µm which particles have been produced by mixing a solution of a matrix material, prepared by dissolving a polymer and/or polymerizable monomers in an aqueous solvent for the matrix material and addition of a water-soluble emulsifier, with a solid cavity generating compound to make a suspension of the solid compound in the solution; forming particles of the polymer matrix by dispersion of the suspension in a water-insoluble dispersion medium containing a water-insoluble emulsifier and solidifying the suspension droplets obtained in the medium; and washing out the solid cavity generating compound from the particles.

2. Macroporous particles to be used as ion exchanger in gel filtration, hydrophobic chromatography, affinity chromatography or as microcarriers in cell cultivation processes, characterized in that they have a particle size of 10-500 µm and consist of a polymer matrix enclosing a large number of cavities having a diameter of 1-50 µm which particles have been produced by mixing a solution of a matrix material, prepared by dissolving a polymer and/or polymerizable monomers in an aqueous solvent for the matrix material and addition of a water-soluble emulsifier, with a water-insoluble dispersion medium containing a water-insoluble emulsifier to make a dispersion of the medium in the solution; forming particles of the polymer matrix by dispersing the dispersion in a further amount of the dispersion medium and solidifying the dispersion droplets obtained in the medium; and washing out the dispersion medium from the particles.

3. Particles according to Claim 1 or 2, characterized in that the particles have a size of approximately 200 µm and that the cavities have a diameter of approximately 10-20 µm.

4. Particles according to any of Claims 1-3 , characterized in that the polymer matrix comprises a protein, a polysaccharide or a synthetic polymer.

5. A method of producing macroporous particles to be used as ion exchanger in gel filtration, hydrophobic chromatography, affinity chromatography or as microcarriers in cell cultivation processes, having a particle size of 10-500 µm and comprising a polymer matrix which encloses a large number of cavities of a diameter of 1-50 µm, characterized in
(A) dissolving a matrix material, selected from a water-soluble polymer and/or polymerizable monomers, in an aqueous solvent for the matrix material;
(B) adding a water-soluble emulsifier;
(C) forming cavity containing droplets of the matrix material by:
a) adding a liquid cavity generating material consisting of a water-insoluble dispersion medium containing a water-insoluble emulsifier which dissolves in the dispersion medium, while stirring the system until the dispersion medium forms droplets in the aqueous solution and an emulsion is obtained; and
b) adding additional dispersion medium to saturate the aqueous solution with the dispersion medium and to form droplets of the emulsion in the dispersion medium;
D) solidifying the dispersed droplets by cooling, covalent crosslinking or by polymerizitation to form beads of the polymer matrix each bead having a large number of pores containing the dispersion medium ; and
E) washing out the dispersion medium from the pores of the beads to form the macroporous particles with a large number of cavities.

6. A method of producing macroporous particles to be used as ion exchanger in gel filtration, hydrophobic chromatography, affinity chromatography or as microcarriers in cell cultivation processes, having a particle size of 10-500 µm and comprising a polymer matrix which encloses a large number of cavities of a diameter of 1-50 µm, characterized in
(A) dissolving a matrix material, selected from a water-soluble polymer and/or polymerizable monomers, in an aqueous solvent for the matrix material;
(B) adding a water-soluble emulsifier;
(C) forming cavity containing droplets of the matrix material by:
a) adding a solid cavity generating compound which is insoluble in the aqueous solution while stirring the system to form a suspension of the cavity generating compound in the aqueous solution; and
b) adding the suspension to a water-insoluble dispersion medium containing a water-insoluble emulsifier which dissolves in the dispersion medium while stirring to form a dispersion of droplets of the suspension in the dispersion medium;
D) solidifying the dispersed droplets by cooling, covalent crosslinking or by polymerizitation to form beads of the polymer matrix each bead having a large number of pores containing the solid cavity generating compound; and
E) washing out the solid cavity generating compound from the pores of the beads to form the macroporous particles with a large number of cavities.

7. A method according to Claim 5 or 6, characterized in that the water-insoluble dispersion medium is an organic solvent, a vegetable oil or a mineral oil.

8. A method according to claim 6, characterized in that the solid cavity generating compound is calcium carbonate.

9. A method according to any of Claims 5-8, characterized in binding negative groups, positive groups, hydrophobic groups or affinity ligands to the matrix of the produced particles.

10. A method according to any of Claims 5-9, characterized in selecting the matrix material from the group of proteins, polysaccarides or polymerizable monomers.

11. The use of macroporous particles according to Claim 1 or 2 with a particle size of 10-500 µm and comprising a polymer matrix enclosing a large number of cavities having a diameter of 1-50 µm as an ion exchanger in gel filtration, hydrophobic chromatography, affinity chromatography or as microcarriers in cell cultivation processes.

## Patentansprüche

1. Makroporöse Partikel zur Verwendung als Ionenaustauscher bei Gelfiltration, hydrophober Chromatographie, Affinitätschromatographie oder als Mikroträger bei Zellkultivierungsprozessen, dadurch gekennzeichnet, daß sie eine Partikelgröße von 10 bis 500 µm aufweisen und aus einer Polymermatrix bestehen, die eine große Zahl von Hohlräumen mit einem Durchmesser von 1 bis 50 µm umfaßt, wobei die Partikel erzeugt worden sind durch Mischen einer Lösung eines Matrixmaterials, welches durch Lösen eines Polymers und/oder polymerisierbarer Monomere in einem wässrigen Lösungsmittel für das Matrixmaterial und durch Zugabe eines wasserlöslichen Emulgators hergestellt ist, mit einer festen, hohlraumerzeugenden Verbindung, um eine Suspension der festen Verbindung in der Lösung herzustellen; durch Bilden von Partikeln der Polymermatrix durch Dispersion der Suspension in einem wasserunlöslichen Dispersionsmedium, das einen wasserunlöslichen Emulgator enthält, und durch Verfestigen der in dem Medium gewonnenen Suspensionströpfchen; und durch Auswaschen der festen, hohlraumerzeugenden Verbindung aus den Partikeln.

2. Makroporöse Partikel zur Verwendung als Ionenaustauscher bei Gelfiltration, hydrophober Chromatographie, Affinitätschromatographie oder als Mikroträger bei Zellkultivierungsprozessen, dadurch gekennzeichnet, daß sie eine Partikelgröße von 10 bis 500 µm aufweisen und aus einer Polymermatrix bestehen, die eine große Zahl von Hohlräumen mit einem Durchmesser von 1 bis 50 µm umfaßt, wobei die Partikel erzeugt worden sind durch Mischen einer Lösung eines Matrixmaterials, das durch Lösen eines Polymers und/oder polymerisierbarer Monomere in einem wässrigen Lösungsmittel für das Matrixmaterial und durch Zugabe eines wasserlöslichen Emulgators hergestellt ist, mit einem wasserunlöslichen Dispersionsmedium, das einen wasserunlöslichen Emulgator enthält, um eine Dispersion des Mediums in der Lösung herzustellen; durch Bilden von Partikeln der Polymermatrix durch Dispergieren der Dispersion in einer weiteren Menge des Dispersionsmediums und durch Verfestigen der in dem Medium gewonnenen Dispersionströpfchen; und durch Auswaschen des Dispersionsmediums aus den Partikeln.

3. Partikel gemäß Anspruch 1 oder 2, dadurch gekennzeichnet, daß die Partikel eine Größe von etwa 200 µm aufweisen und daß die Hohlräume einen Durchmesser von etwa 10 bis 20 µm aufweisen.

4. Partikel gemäß einem der Ansprüche 1 bis 3, dadurch gekennzeichnet, daß die Polymermatrix ein Protein, ein Polysaccharid oder ein synthetisches Polymer enthält.

5. Verfahren zum Herstellen makroporöser Partikel, die als Ionenaustauscher bei Gelfiltration, hydrophober Chromatographie, Affinitätschromatographie oder als Mikroträger bei Zellkultivierungsprozessen verwendet werden, wobei sie eine Partikelgröße von 10 bis 500 µm aufweisen und eine Polymermatrix enthalten, die eine große Zahl von Hohlräumen eines Durchmessers von 1 bis 50 µm umfaßt, gekennzeichnet durch
(A) Lösen eines Matrixmaterials, das aus einem wasserlöslichen Polymer und/oder polymerisierbaren Monomeren ausgewählt ist, in einem wässrigen Lösungsmittel für das Matrixmaterial;
(B) Hinzugeben eines wasserlöslichen Emulgators;
(C) Bilden von hohlraumenthaltenden Tröpfchen des Matrixmaterials durch:
a) Hinzugeben eines flüssigen, hohlraumerzeugenden Materials, das aus einem wasserunlöslichen Dispersionsmedium besteht, das einen wasserunlöslichen Emulgator enthält, der sich in dem Dispersionsmedium löst, während das System gerührt wird, bis das Dispersionsmedium Tröpfchen in der wässrigen Lösung bildet und eine Emulsion gewonnen wird; und
b) Hinzugeben von zusätzlichem Dispersionsmedium, um die wässrige Lösung mit dem Dispersionsmedium zu sättigen und um Tröpfchen der Emulsion in dem Dispersionsmedium zu bilden;
(D) Verfestigen der dispergierten Tröpfchen durch Abkühlen, kovalente Vernetzung oder durch Polymerisation, um Kügelchen der Polymermatrix zu bilden, wobei jedes Kügelchen eine große Zahl von Poren aufweist, die das Dispersionsmedium enthalten; und
(E) Auswaschen des Dispersionsmediums aus den Poren der Kügelchen, um die makroporösen Partikel mit einer großen Zahl von Hohlräumen zu bilden.

6. Verfahren zum Herstellen makroporöser Partikel, die als Ionenaustauscher bei Gelfiltration, hydrophober Chromatographie, Affinitätschromatographie oder als Mikroträger bei Zellkultivierungsprozessen verwendet werden, wobei sie eine Partikelgröße von 10 bis 500 µm aufweisen und eine Polymermatrix enthalten, die eine große Zahl von Hohlräumen eines Durchmessers von 1 bis 50 µm umfaßt, gekennzeichnet durch
(A) Lösen eines Matrixmaterials, das aus einem wasserlöslichen Polymer und/oder polymerisierbaren Monomeren ausgewählt ist, in einem wässrigen Lösungsmittel für das Matrixmaterial;
(B) Hinzugeben eines wasserlöslichen Emulgators;
(C) Bilden von hohlraumenthaltenden Tröpfchen des Matrixmaterials durch:
a) Hinzugeben einer festen, hohlraumerzeugenden Verbindung, die in der wässrigen Lösung unlöslich ist, während das System gerührt wird, um eine Suspension der hohlraumerzeugenden Verbindung in der wässerigen Lösung zu bilden; und
b) Hinzugeben der Suspension zu einem wasserunlöslichen Dispersionsmedium, das einen wasserunlöslichen Emulgator enthält, der sich in dem Dispersionsmedium löst, während gerührt wird, um eine Dispersion von Tröpfchen der Suspension in dem Dispersionsmedium zu bilden;
(D) Verfestigen der dispergierten Tröpfchen durch Abkühlen, kovalente Vernetzung oder durch Polymerisation, um Kügelchen der Polymermatrix zu bilden, wobei jedes Kügelchen eine große Zahl von Poren aufweist, die die feste, hohlraumerzeugende Verbindung enthalten; und
(E) Auswaschen der festen, hohlraumerzeugenden Verbindung aus den Poren der Kügelchen, um die makroporösen Partikel mit einer großen Zahl von Hohlräumen zu bilden.

7. Verfahren gemäß Anspruch 5 oder 6, dadurch gekennzeichnet, daß das wasserunlösliche Dispersionsmedium ein organisches Lösungsmittel, ein pflanzliches Öl oder ein mineralisches Öl ist.

8. Verfahren gemäß Anspruch 6, dadurch gekennzeichnet, daß die feste, hohlraumerzeugende Verbindung Calciumcarbonat ist.

9. Verfahren gemäß einem der Ansprüche 5 bis 8, gekennzeichnet durch das Binden von negativen Gruppen, positiven Gruppen, hydrophoben Gruppen oder Affinitätsliganden an die Matrix der erzeugten Partikel.

10. Verfahren gemäß einem der Ansprüche 5 bis 9, gekennzeichnet durch das Auswählen des Matrixmaterials aus der Gruppe von Proteinen, Polysacchariden oder polymerisierbaren Monomeren.

11. Verwendung von makroporösen Partikeln gemäß Anspruch 1 oder 2, die eine Partikelgröße von 10 bis 500 µm aufweisen und eine Polymermatrix enthalten, die eine große Zahl von Hohlräumen umfaßt, die einen Durchmesser von 1 bis 50 µm aufweisen, als ein Ionenaustauscher bei der Gelfiltration, der hydrophoben Chromatographie, der Affinitätschromatographie oder als Mikroträger bei Zellkultivierungsprozessen.

## Revendications

1. Particules macroporeuses destinées à être utilisées comme échangeur d'ions dans la filtration sur gel, la chromatographie hydrophobe, la chromatographie par affinité ou comme microporteurs dans les processus de culture de cellules, caractérisées en ce qu'elles ont une taille de particule de 10-500 µm et en ce qu'elles consistent en un réseau de base polymère renfermant un grand nombre de cavités ayant un diamètre de 1-50 µm, ces particules ayant été produites par : le mélange d'une solution d'une matière du réseau de base, préparée par dissolution d'un polymère et/ou de monomères polymérisables dans un solvant aqueux pour la matière du réseau de base et par addition d'un émulsifiant soluble dans l'eau, avec un composé solide générant des cavités pour obtenir une suspension du composé solide dans la solution; la formation de particules du réseau de base polymère par dispersion de la suspension dans un agent de dispersion insoluble dans l'eau contenant un émulsifiant insoluble dans l'eau, ainsi que la solidification des gouttelettes de la suspension obtenues dans l'agent de dispersion; et le lavage du composé solide générant des cavités afin de l'éliminer des particules.

2. Particules macroporeuses destinées à être utilisées comme échangeur d'ions dans la filtration sur gel, la chromatographie hydrophobe, la chromatographie par affinité ou comme microporteurs dans les processus de culture de cellules, caractérisées en ce qu'elles ont une taille de particule de 10-500 µm et en ce qu'elles consistent en un réseau de base polymère renfermant un grand nombre de cavités ayant un diamètre de 1-50 µm, ces particules ayant été produites par: le mélange d'une solution d'une matière du réseau de base, préparée par dissolution d'un polymère et/ou de monomères polymérisables dans un solvant aqueux pour la matière du réseau de base et par addition d'un émulsifiant soluble dans l'eau, avec un agent de dispersion insoluble dans l'eau contenant un émulsifiant insoluble dans l'eau pour obtenir une dispersion de l'agent de dispersion dans la solution; la formation de particules du réseau de base polymère par dispersion de la dispersion dans une quantité additionnelle de l'agent de dispersion, ainsi que la solidification des gouttelettes de la dispersion obtenues dans l'agent de dispersion; et le lavage de l'agent de dispersion afin de l'éliminer des particules.

3. Particules selon la revendication 1 ou 2, caractérisées en ce que les particules ont une taille d'approximativement 200 µm et en ce que les cavités ont un diamètre d'approximativement 10-20 µm.

4. Particules selon l'une quelconque des revendications 1-3, caractérisées en ce que le réseau de base polymère comprend une protéine, un polysaccharide ou un polymère synthétique.

5. Procédé de production de particules macroporeuses destinées à être utilisées code échangeur d'ions dans la filtration sur gel, la chromatographie hydrophobe, la chromatographie par affinité ou comme microporteurs dans les processus de culture de cellules, ayant une taille de particule de 10-500 µm et comprenant un réseau de base polymère qui renferme un grand nombre de cavités d'un diamètre de 1-50 µm, caractérisé par :
(A) la dissolution d'une matière du réseau de base, choisie parmi un polymère soluble dans l'eau et/ou des monomères polymérisables, dans un solvant aqueux pour la matière du réseau de base;
(B) l'addition d'un émulsifiant soluble dans l'eau;
(C) la formation de gouttelettes de la matière du réseau de base contenant des cavités par :
a) addition d'une matière liquide générant des cavités, consistant en un agent de dispersion insoluble dans l'eau contenant un émulsifiant insoluble dans l'eau qui se dissout dans l'agent de dispersion, tout en agitant le système jusqu'à ce que l'agent de dispersion forme des gouttelettes dans la solution aqueuse et qu'une émulsion soit obtenue; et
b) addition d'agent de dispersion additionnel pour saturer la solution aqueuse avec l'agent de dispersion et pour former des gouttelettes de l'émulsion dans l'agent de dispersion;
(D) la solidification des gouttelettes dispersées par refroidissement, par réticulation covalente ou par polymérisation pour former des perles du réseau de base polymère, chaque perle ayant un grand nombre de pores contenant l'agent de dispersion; et
(E) le lavage de l'agent de dispersion afin de l'éliminer des pores des perles pour former les particules macroporeuses avec un grand nombre de cavités.

6. Procédé de production de particules macroporeuses destinées à être utilisées conune échangeur d'ions dans la filtration sur gel, la chromatographie hydrophobe, la chromatographie par affinité ou comme microporteurs dans les processus de culture de cellules, ayant une taille de particule de 10-500 µm et comprenant un réseau de base polymère qui renferme un grand nombre de cavités d'un diamètre de 1-50 µm, caractérisé par :
(A) la dissolution d'une matière du réseau de base, choisie parmi un polymère soluble dans l'eau et/ou des monomères polymérisables, dans un solvant aqueux pour la matière du réseau de base;
(B) l'addition d'un émulsifiant soluble dans l'eau;
(C) la formation de gouttelettes de la matière du réseau de base contenant des cavités par :
a) addition d'un composé solide générant des cavités, qui est insoluble dans la solution aqueuse, tout en agitant le système pour former une suspension du composé générant des cavités dans la solution aqueuse; et
b) addition de la suspension à un agent de dispersion insoluble dans l'eau contenant un émulsifiant insoluble dans l'eau, qui se dissout dans l'agent de dispersion, tout en agitant pour former une dispersion de gouttelettes de la suspension dans l'agent de dispersion;
(D) la solidification des gouttelettes dispersées par refroidissement, par réticulation covalente ou par polymérisation pour former des perles du réseau de base polymère, chaque perle ayant un grand nombre de pores contenant le composé solide générant des cavités; et
(E) le lavage du composé solide générant des cavités afin de l'éliminer des pores des perles, pour former les particules macroporeuses avec un grand nombre de cavités.

7. Procédé selon la revendication 5 ou 6, caractérisé en ce que l'agent de dispersion insoluble dans l'eau est un solvant organique, une huile végétale ou une huile minérale.

8. Procédé selon la revendication 6, caractérisé en ce que le composé solide générant des cavités est le carbonate de calcium.

9. Procédé selon l'une quelconque des revendications 5-8, caractérisé par la liaison de groupes négatifs, de groupes positifs, de groupes hydrophobes ou de ligands à affinité avec le réseau de base des particules produites.

10. Procédé selon l'une quelconque des revendications 5-9, caractérisé par le choix de la matière du réseau de base à partir du groupe constitué par des protéines, des polysaccharides ou des monomères polymérisables.

11. Utilisation de particules macroporeuses selon la revendication 1 ou 2, présentant une taille de particule de 10-500 µm et comprenant un réseau de base polymère renfermant un grand nombre de cavités ayant un diamètre de 1-50 µm, comme échangeur d'ions dans la filtration sur gel, la chromatographie hydrophobe, la chromatographie par affinité ou comme microporteurs dans les processus de culture de cellules.
